# EUROPEAN PATENT APPLICATION

(11) **EP 4 098 180 A1**
(43) Date of publication of application: **07.12.2022**
(21) Application number: 21747737.1
(22) Date of filing: 29.01.2021
(51) Int. Cl.: A61B 5/01, A61B 6/00

(54) **MOBILE SYSTEM AND AUXILIARY METHOD FOR EVALUATING THERMOGRAPHIC BREAST IMAGES**

(30) Priority: 30.01.2020 BR 102020002019
(71) Applicant: Termo Health Tecnologia Ltda, 01452-000 São Paulo (BR)
(72) Inventor: DE SOUTO, Fellipe Belmino, 05782-460 São Paulo (BR)
(74) Representative: Grünecker Patent- und Rechtsanwälte PartG mbB
(86) International application number: PCT/BR2021/050043
(87) International publication number: WO 2021/151186

(57) **Abstract**

The present invention describes an architecture for a mobile system and process for evaluating breast thermographic images. Specifically, the present invention comprises a system capable of analyzing and evaluating breast thermal images of a patient captured by a mobile device connected to a thermal imager, wherein the analysis returns to an auxiliary index, which is evaluated through an artificial intelligence tool, so that a health professional can make a decision, being able to show a diagnosis or lead the patient to more specific exams. The present invention is in the fields of medicine and computer engineering.

## Description

### Field of the Invention

The present invention describes an architecture for a mobile system and process for evaluating breast thermographic images. More specifically, the present invention comprises a system capable of analyzing and evaluating breast thermal images of a patient and returning an auxiliary index, evaluated through an artificial intelligence tool, so that a health professional can make a decision. The present invention is in the fields of medicine and computer engineering.

### Background of the Invention

According to data from the Brazil's National Cancer Institute, Brazil had 57,000 new cases of breast cancer in 2016. In addition, breast cancer is the second most fatal type of cancer in Brazil and has an annual growth rate of 3.75%. Furthermore, 90% cases have no genetic relationship and have 88.3% survival when diagnosed early. In this sense, actions to prevent breast cancer are essential for increasing survival rates.

In addition, the more advanced disease stages make the treatment cost 60% to 80% more expensive. On the other hand, Brazil has only 25% the necessary structure to serve the population and 15% this structure is not available due to technical problems or lack of professionals. Regarding this, only 11% the mammographies that should have been done in 2018 were performed by the Brazil's Health Care System.

Thus, the current ways of prevention, early diagnosis and care for people with breast cancer are difficult to carry out, since there is currently a lack of structures and equipment for mammography exams necessary to meet the growing number of breast cancer cases. Also, the long waiting queues for medical appointments and mammograms make it difficult to correctly prevent and early diagnosis of the disease.

In addition, mammography exams currently performed by a mammography device cause discomfort and pain to the patient. Moreover, the examination is performed only in places where mammography equipment is available and is not easily accessible.

Therefore, it is urgent that ways and actions are taken so that the prevention and early diagnosis of breast cancer are done quickly, simply, accessible to the population and capable of anticipating the diagnosis before the first clinical symptoms.

In the prior art search in scientific and patent literature, the following documents were found dealing with the subject matter:

The document WO2019241380 refers to a method for detecting cancerous tumors by means of artificial intelligence trained to analyze thermal images, so that these thermal images can be obtained through a smartphone. The solution proposed by WO2019241380 does not propose a system architecture that is mobile and easily implementable, nor does it propose an index to aid in medical diagnosis.

The article entitled "A portable breast cancer detection system based on smartphone with infrared camera" by Jian Ma, et al, describes a smartphone-based breast cancer detection system with infrared thermal camera. The proposed system performs the following steps: obtaining the thermal image of the breasts; grayscale pre-processing; the areas of interest are segmented; the right breast is flipped for comparison with the left breast; features such as average temperature, contrast, entropy and energy are extracted through an algorithm and then a trained neural network classifies the images, based on these parameters, as healthy or with the possibility of cancer. However, such article fails to expose this technology being implemented in a mobile and simplified architecture, to make it impossible to deploy in places of easy access to several users.

The article entitled "Thermal Infrared Image Analysis for Breast Cancer Detection" by Sedong Min, et al, deals with a way of detecting breast cancer through infrared thermal analysis by a thermal camera that can be attached to a smartphone, according to the author, wherein the thermal images are preprocessed, leaving the images in grayscale. To compare inconsistencies between the right breast and the left breast, Shannon's entropy is used as an analysis parameter, wherein in healthy breasts, entropies tend to be equal in both breasts, thus using a neural network to classify the images. According to the author, in breasts where there was a major difference in entropy, there was an occurrence of cancer. However, this article fails to expose this technology being implemented in a mobile and simplified architecture, to make it impossible to deploy in places of easy access to several users.

The article entitled "Non-contact Noninvasive Early Breast-Cancer Screening Device" by Bilal Majeed et al, describes the use of a thermal camera for breast cancer identification, where the image is extracted from the camera, preprocessed in grayscale and filters are applied to select the area of interest. Neural networks are trained to discriminate between benign and malignant cancer cells through the values of neighboring pixels. The classification used by the author differs from the one proposed in the present invention, regarding the analysis of thermal images. Additionally, the article fails to expose this technology being implemented in a mobile and simplified architecture, to make it unfeasible to deploy in places with easy access to several users.

The document KR101889725 describes a method for diagnosing or prognosticating malignant tumors by comparing images by convolutional neural networks, also citing the generation of a tumor proliferation rate (by the amount and location of mitotic cells). The training of neural networks by breast images is cited, however these images are scanned tissue slides, not citing the use of thermal analysis nor the use in smartphones. Additionally, this solution does not allow implementation in simplified architectures for mobile deployment in locations that are easily accessible for users and patients.

Thus, from what can be seen from the researched literature, no documents were found anticipating or suggesting the teachings of the present invention, so that the solution proposed herein has novelty and inventive step compared to the prior art.

### Summary of the Invention

In this way, the present invention solves the prior art problems from a concept of mobility in the breast cancer exams performance, taking this possibility to places where there is no mammography device or there are long queues for exams, working, for example, as a way to screen or prioritize calls. For this, the present invention has a simplified architecture that is easy to implement in these places, so that the architecture proposed herein comprises a mobile electronic device connected to a thermal imager, wherein the breast thermal image captured from the patient is sent to a server capable of analyzing and evaluating the image, thus promoting an index of suspicion to assist a health professional. With that, from this index, the health professional can carry out the diagnosis starting from the result obtained by this index, where the professional can make the decision to lead the patient to carry out more specific exams or even indicate, based on his/her experience, the existence of a tumor or not.

In a first object, the present invention shows an auxiliary mobile system for evaluating breast thermographic images that comprises: a mobile electronic device connected with at least one thermal imager, the mobile electronic device being provided with at least one electronic application (3) embedded provided with an interface; an intermediate module (2) communicating with the electronic application (3), the intermediate module (2) being provided with a data driver, in which the intermediate module (2) receives thermal image data from the electronic application (3); and a server (1), communicating with the intermediate module (2), provided with an artificial intelligence tool and a data repository, wherein the intermediate module (2) sends the captured thermal image data to the server (1); and the artificial intelligence tool receives the thermal image data and returns an index of suspicion, which is received by the intermediate module (2) and directed to the electronic application (3) embedded in the mobile electronic device; being the artificial intelligence tool previously trained and fed with breast thermographic images.

In a second object, the present invention shows an auxiliary process for evaluating breast thermographic images comprising the steps of: collecting at least one breast thermographic image by an electronic application (3) embedded in a mobile electronic device; sending the breast thermographic image to a server (1), which is provided with an artificial intelligence tool that evaluates the breast thermographic image and returns an index of suspicion; and receiving the index of suspicion by the electronic application (3) and availability of the index of suspicion in the interface of the electronic application (3).

These and other objects of the invention will be immediately appreciated by those skilled in the art and will be described in detail below.

### Brief Description of the Drawings

The following figures are showed:
Fig. 1 shows an embodiment of the action flowchart.
Fig. 2 shows an embodiment of the system class diagram.
Fig. 3 shows an embodiment of the system architecture.
Fig. 4 shows an embodiment of the system architecture.
Fig. 5 shows an embodiment of the home screen of the mobile device platform.
Fig. 6 shows an embodiment of the application's pre-exam screen.
Fig. 7 shows an embodiment of the front image capture screen.
Fig. 8 shows an embodiment of the left side image capture screen.
Fig. 9 shows an embodiment of the right-side image capture screen.
Fig. 10 shows an embodiment of the image review screen.
Fig. 11 shows an embodiment of the result screen.
Fig. 12 shows an embodiment of the profile screen.
Fig. 13 shows an embodiment of the exam history screen.
Fig. 14 shows an embodiment of the exam detail screen.
Fig. 15 shows an embodiment of the thermal imager.
Fig. 16 shows an embodiment of the thermal imager connected with a mobile electronic device through physical connection.
Fig. 17 shows an embodiment of the thermal imager.
Fig. 18 shows an embodiment of the thermal imager connected with a mobile electronic device through physical connection.
Fig. 19 shows an embodiment of the thermal imager.
Fig. 20 shows an embodiment of the login screen, which shows the connection of the thermal imager to the mobile electronic device.

### Detailed Description of the Invention

The descriptions that follow are showed by way of example and do not limit the scope of the invention and will make the object of the present patent application more clearly understood.

In a first object, the present invention shows an auxiliary mobile system for evaluating breast thermographic images that comprises: a mobile electronic device connected with at least one thermal imager, the mobile electronic device being provided with at least one electronic application (3) embedded provided with an interface; an intermediate module (2) communicating with the electronic application (3), the intermediate module (2) being provided with a data driver, in which the intermediate module (2) receives thermal image data from the electronic application (3); and a server (1), communicating with the intermediate module (2), provided with an artificial intelligence tool and a data repository, wherein the intermediate module (2) sends the captured thermal image data to the server (1); and the artificial intelligence tool receives the thermal image data and returns an index of suspicion, which is received by the intermediate module (2) and directed to the electronic application (3) embedded in the mobile electronic device; being the artificial intelligence tool previously trained and fed with breast thermographic images.

In one embodiment, the mobile electronic device is a smartphone, cell phone, notebook, etc., which may be intrinsically provided with a thermal image sensor. In one embodiment, the thermal imager is a thermal image sensor module connectable to the mobile electronic device. For purposes of exemplification, the imager is commonly known as a thermal camera, and this term may be used/repeated throughout the present application, without limiting the scope of the invention.

In one embodiment, the thermal camera is connected to the mobile electronic device via wireless connection, e.g., Wi-Fi, Bluetooth, etc. In another embodiment, the thermal camera is connected to the mobile electronic device via physical connection (cables, connectors, etc.).

For the purposes of the present invention, the electronic application (3) is any software (or firmware) capable of being implemented in the electronic device, generating a user access interface, in such a way that the operation is performed by the user. In one embodiment, the electronic application (3) comprises an interface provided with a form to obtain statistical data from the patient (not necessarily personal data), in addition to having a tool that makes it possible to view the image captured by the thermal camera.

In a further embodiment, the electronic application (3) comprises a mask to assist in framing the thermal image captured by the thermal camera. Furthermore, in this embodiment, the electronic application (3) indicates the required or preferred positions for obtaining the thermal images.

The intermediate module (2) can be understood as any software structure/component mediating and targeting information between the electronic application (3) and the server (1), which may be implemented in the mobile electronic device, in the electronic application (3), on the server (1), on a web page, etc. In one embodiment, the intermediate module (2) is an API implemented locally on the mobile electronic device. In another embodiment, the intermediate module (2) is an API that runs in a Web environment. In another embodiment, the intermediate module (2) is an API that runs on the server (1) itself.

Thus, the intermediate module (2) is responsible for directing the data from the breast thermographic image to the server (1) and, soon after receiving the response from the server with the index of suspicion calculated, the intermediate module (1) directs the index both for the electronic application and for the repository of the server (1).

In one embodiment, the breast thermographic image data results from a conversion of the breast thermographic image into a data string (character chain). This conversion can be performed by the mobile electronic device, by the electronic application (3) or by the intermediate module (2).

In one embodiment, upon receiving the index of suspicion directed by the intermediate module (2), the repository updates a patient history with the index of suspicion obtained and with patient information (not necessarily patient's personal information).

As for the server (1) used in the system architecture, it can be physical or remote, capable of communicating with the electronic application (3). In one embodiment, the server (1) is a cloud operable remote server.

In one embodiment, said artificial intelligence tool is a convolutional neural network. This convolutional neural network is previously trained and fed with breast thermography images obtained, for example, in exams performed by people skilled in the subject. Based on this, the thermographic images were input into the neural network with the respective diagnostic results linked, for example, a sequence of thermal mammographic images wherein the cancer was diagnosed by a physician.

From this, the convolutional neural network can analyze captured thermographic images and show a probability that the patient will develop a tumor related to breast cancer.

In a second object, the present invention shows an auxiliary process for evaluating breast thermographic images comprising the steps of: collecting at least one breast thermographic image by an electronic application (3) embedded in a mobile electronic device; sending the breast thermographic image to a server (1), which is provided with an artificial intelligence tool that evaluates the breast thermographic image and returns an index of suspicion; and receiving the index of suspicion by the electronic application (3) and availability of the index of suspicion in the interface of the electronic application (3).

The step of collecting a breast thermographic image by the electronic application (3) can be performed: i) upon loading a breast thermographic image previously acquired and/or stored eventually in a database; or ii) by means of a thermal camera which, when capturing the image, forwards it to the electronic application (3).

In one embodiment, a previous step of capturing the breast thermographic image is performed by a thermal imager connected to the mobile electronic device, wherein the step of capturing the breast thermographic image comprises a sub-step of framing the image from a support mask implemented in the electronic application interface (3).

In one embodiment, the auxiliary process of the present invention is implemented in the system herein described.

Thus, starting from the previous embodiment - referring to the implementation in the system, the step of sending the breast thermographic image to a server (1) comprises the sub-steps: conversion of the breast thermographic image into a data string on the mobile electronic device; and receiving an evaluation request by an intermediate module (2), communicating with the electronic application (3) and with the server (1), wherein the intermediate module (2) receives the data from the breast thermographic image and forwards it to the server (1). Said intermediate module (2) is responsible for receiving the index of suspicion evaluated by the artificial intelligence tool of the server (1) and sending it to the electronic application (3).

Additionally, a step of sending and updating patient history data is performed, wherein the intermediate module (2) sends patient information and/or index of suspicion to the repository of the server (1).

In one embodiment, the artificial intelligence tool is a convolutional neural network previously trained and fed with breast thermography images and the respective diagnostic results previously identified. Based on this, the convolutional neural network performs the analysis step of the thermal image captured by the thermal camera and probabilistically evaluates the chances of the patient developing a breast cancer-related tumor.

In one embodiment, prior to the evaluation performed by the artificial intelligence tool, a pre-processing is performed on the breast thermographic image. Said pre-processing is performed by dividing the image into two halves, such as a horizontal flip technique is performed on the first half and the resulting is compared with the second half, to analyze the pattern of colors of the thermographic image, so that to identify similarities and/or disparities in patterns.

In one embodiment, the result of the analysis follows, in addition to the index of suspicion, a recommendation to seek or not to seek a specialized physician in the field.

In one embodiment, the evaluated thermal image is fed back into the convolutional neural network, together with a diagnosis/opinion of a specialized physician, for training and improving the analysis and evaluation performed by the convolutional neural network.

Thus, in view of the objects herein showed that materialize the proposed concept, it is possible to state that what is exposed in this document is advantageous compared to current technologies performing mammograms, since current mammography equipment is severely large and incapable of becoming mobile, in addition to there is an extreme demand. With this technology, on the other hand, it is possible to provide an index to assist a qualified health professional/specialist in the diagnosis of breast cancer, so that he/she can make the decision to refer the patient for more specific exams or even take the decision regarding the diagnosis of the existence or not of the breast tumor. The technology proposed in the present invention makes it possible to take this type of exam to places where there is no mammography device or there are long waiting queues for exams, which can work, for example, as a way of executing a triage or prioritizing care.

### Example 1 - System Architecture

The examples shown herein are intended only to exemplify one of the numerous ways of performing the invention, however without limiting its scope.

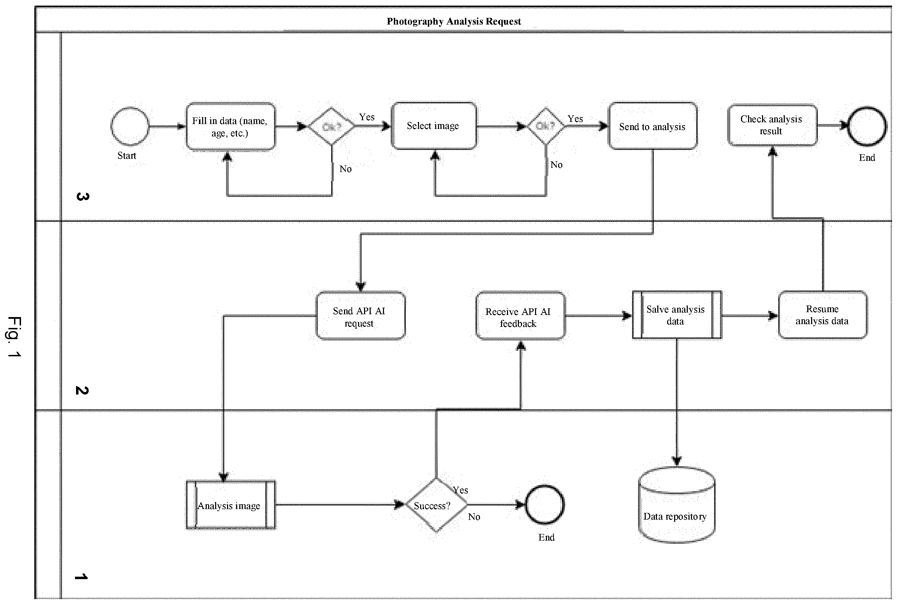

Fig. 1 shows an action flowchart the system performs to request analysis of a patient photograph. Initially, the patient fills in personal data, such as name, age, zip code, etc. Then, the image photographed by the thermal imaging sensor is selected and sent for analysis. Subsequently, the intermediate module (2), in this case an API, performs an image analysis request to the server (1) and then, the thermal image is analyzed by an artificial intelligence tool, which is a convolutional neural network. After the analysis, the API (2) receives the feedback from the server (1) and, from this saves the image analysis data in an analysis repository. Finally, the API receives the analysis status feedback and sends the index of suspicion to the electronic application (3).

The index of suspicion, in this case, is a probability that the patient develops a breast cancer-related tumor based on the analysis of the breast thermographic image.

For this analysis, a pre-processing is performed on the breast thermographic image before being submitted to the convolutional neural network. In this example, a pre-processing algorithm was implemented dividing the image into two halves, starting to work with image A and image B, then the algorithm applies a horizontal flip technique on image A so that it is compared with image B, analyzing the color pattern to identify similarity in colors. If a pattern disparity is detected, it is an indication of an anomaly that should be checked, because according to thermology studies, hyper-radiated areas in the human body indicate a high metabolic activity, which in turn, can indicate an area with an active inflammatory process.

The same process is applied to image B. Then image A is submitted to the process of identifying colors per pixel, assigned a scale from 0 to 1 that considers the body thermal variation from 25 to 36 degrees, wherein 0 is black/blue representing hypo-radiant areas and 1 is red/white representing hyper-radiant areas. In this way, the algorithm extracts the region of interest of the image (ROI).

This decimal scale represents each pixel of the image as a thermal factor, for example, if a pixel is red/white, it can have a value between 0.9 and 1. In this way, it is possible to identify areas with higher concentration of hyper-radiation compared to the same area as image B. The same process is applied to image B.

Therefore, it is possible to identify thermal anomalies that may indicate high metabolic activity where it normally would not be. Then, the image ROI is submitted to the convolutional neural network, making it possible to learn about suspicious or non-suspicious patterns.

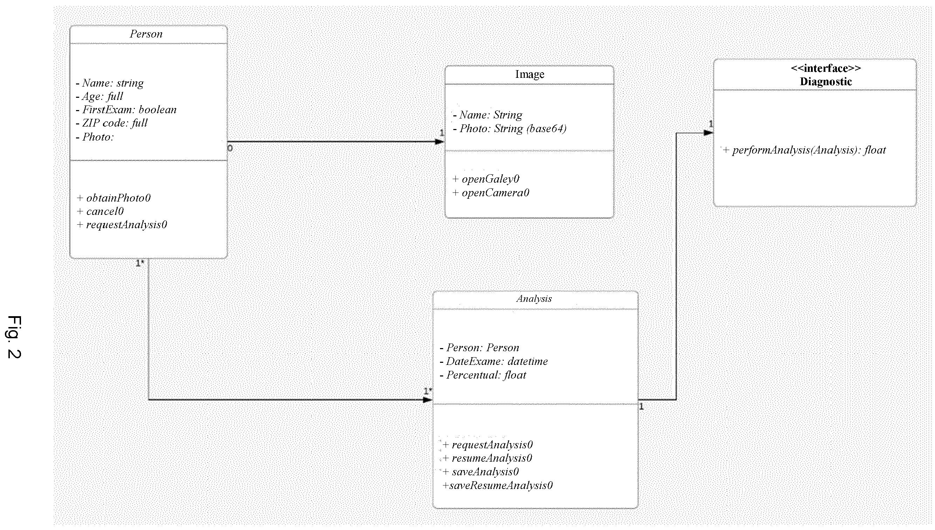

Fig. 2 shows a system class diagram. Within the class called "Person", the system receives the patient's name in a string, the age in integer character, the first exam in Boolean logic, the zip code in integer and the photo in image. Also, this class gets photo, cancels and requests analysis. The "Image" class receives name and image in string, as well as it opens the gallery and opens the thermal camera. In the "Analysis" class, it receives personal data, exam date and percentage, and performs the function of: analysis request, returning analysis, saving analysis and saving analysis return. Finally, in the "Diagnostic Interface" class, it performs the analysis.

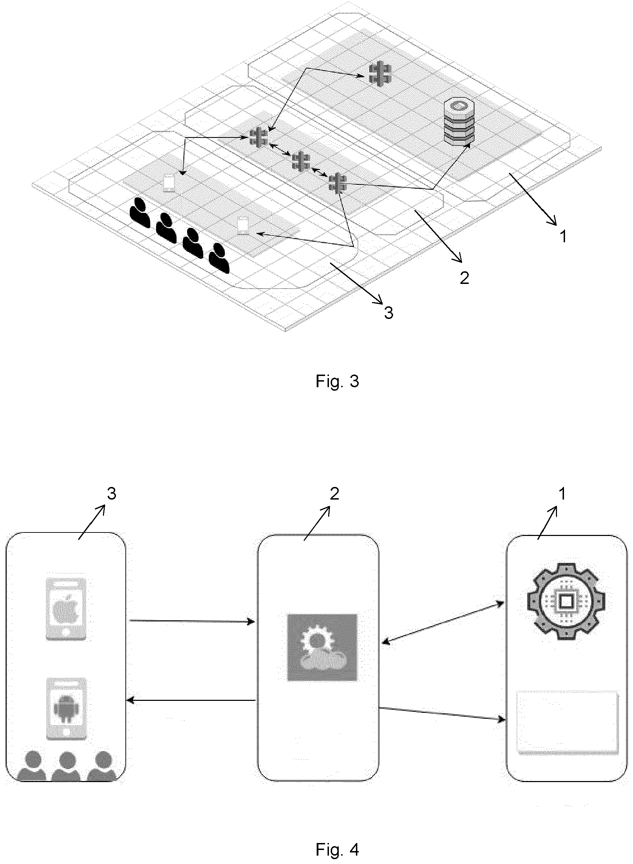

Fig. 3 shows another view of the system architecture. The server (1) comprises image recognition through artificial intelligence and a database that receives data in real time. The intermediary module (2) comprises the programming and development of the functions performed in the system. The mobile device platform (3) - electronic application - comprises the system platform available in the smartphone application store. Said platform (3) is accessible to the patient, where the patient receives the result of the evaluation and analysis of the thermal image.

Fig. 4 also shows another representation of the system architecture. As shown, the intermediate module (2) receives a request from the mobile device platform (3), requests and receives a response from the main module (1), after artificial intelligence analysis, and sends data to the database for storage, as well as shows the analysis feedback to the platform (3).

### Example 2 - App Linda

Thus, in a performance of the invention presented herein, it's shown a sequence of steps that a patient and/or user performs when using the electronic application in the system, and process proposed herein. It is worth noting that both a patient and a healthcare professional can operate these steps in the system.

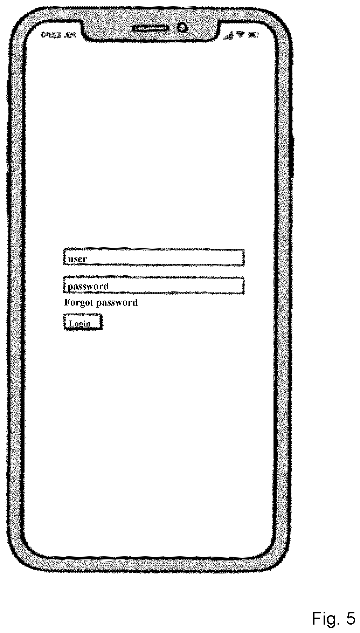

Fig. 5 shows the home screen of platform of the mobile device (3) corresponding to the login screen on the platform. Said platform (3) is a smartphone application.

On the login screen, the previously registered user can log in using their username (e-mail) and password. In case he/she has forgotten his/her access data, he/she can click on "forgot password" where he/she must fill his/her e-mail in a modal box to receive a temporary password. When accessing the application with the temporary password, the user is redirected to the profile screen where he/she can modify the temporary password. The temporary password lasts for 24 hours.

Also, the login screen has "login" integrations - Correct Login, Wrong Login, Temporary Password Login; "forgot password" integrations - Email found password sent, Email not found; and "Password Reset" integrations - Reset error, password changed successfully.

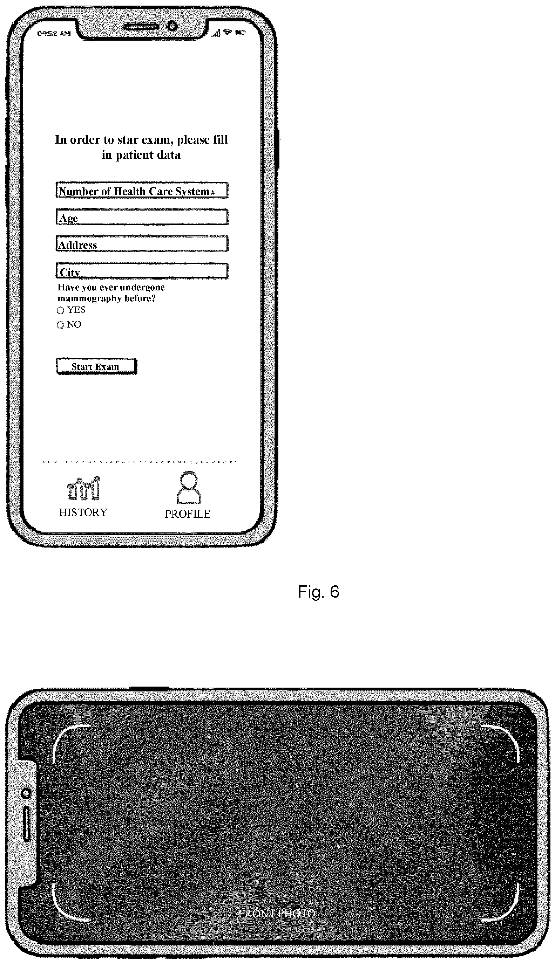

Fig. 6 shows the application pre-exam screen. In the pre-exam screen, the user includes the patient's information before the exam is performed. The user is asked for the following information: Date of Birth, Number of Health Care System, Date of the last period, if she uses hormones, if she has already had a mammogram, and city. Also, the application collects data indirectly to complement patient data, such as: GPS coordinates, temperature of the day and time of the exam. The app saves the information at memory to send the data at the end of the image capture. If there is an error in capturing the image or the app closes, when returning to the exams screen, the data is automatically filled in, making it possible to resume the exam. In addition, the exam screen is the main screen of the app, so the menu is on this screen, containing the items of "History", which directs the user to the performed exam history screen, and the item "Profile" directs the user to the profile screen where the user can view her registration information.

Also, the pre-exam screen has integrations of "Save data offline" - the exam data is saved in the device memory in case an error occurs during the process, making it possible to resume the exam; "Save exam dates" - saves the date and time the exam was taken; and "Save GPS coordinates" - saves the device coordinates for checking, if the device is in the covered area.

In order to perform the exam, in this example, a protocol was proposed to be followed prior to capturing the thermal images. The requirements are described below:
1. Patient must remove the upper part of the clothing covering her breasts (including bra);
2. Patient needs to tie her hair if it is loose;
3. Patient must remove any and all accessories that are around her neck, as well as necklaces, chockers, etc.;
4. Patient must be positioned close to the wall, and the operator must stand at a distance of 40 cm from the tip of her feet to capture the image;
5. Patient should raise her arms, interlacing her fingers behind her head;
6. The room temperature must be between 22° and 23° C;
7. Patient must undergo thermal harmonization, waiting about 15 minutes in the air-conditioned environment;
8. Patient must not have made any physical effort in the last 24 hours;
9. Patient must not be breastfeeding;
10. Patient must not have the flu;
11. Patient must not have a fever or feverishly;
12. Patient must not be in menstrual period;

By checking these requirements, image capture can be performed.

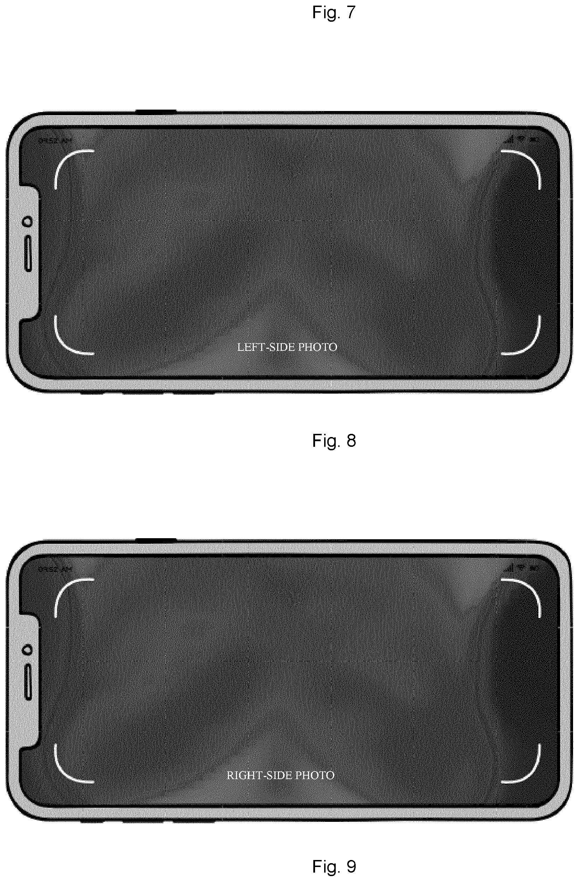

Figs. 7, 8, and 9 show the image capture screens (exam). Fig. 7 shows the frontal image, which is captured to be forwarded to the artificial intelligence tool. Optionally, the system can indicate that side photos of the patient breast are captured. Fig. 8 shows the left side image and Fig. 9 shows the right-side image.

After the user fills in the pre-exam screen with the necessary data and clicks on "start exam", the user is directed to the image capture screen. The screen displays the thermal image captured by the camera and, under the view, there is a mask to help frame the breasts for the capture. Under the mask appear instructions for the user to obtain a better image. Clicking on the screen, the instructions disappear showing the thermal view along with the mask. The mask contains, in addition to the image framing guides, a button to capture the image and the thermal camera battery percentage. If the thermal camera is not connected to the device, a black view appears on the screen with the phrase "Connect thermal camera".

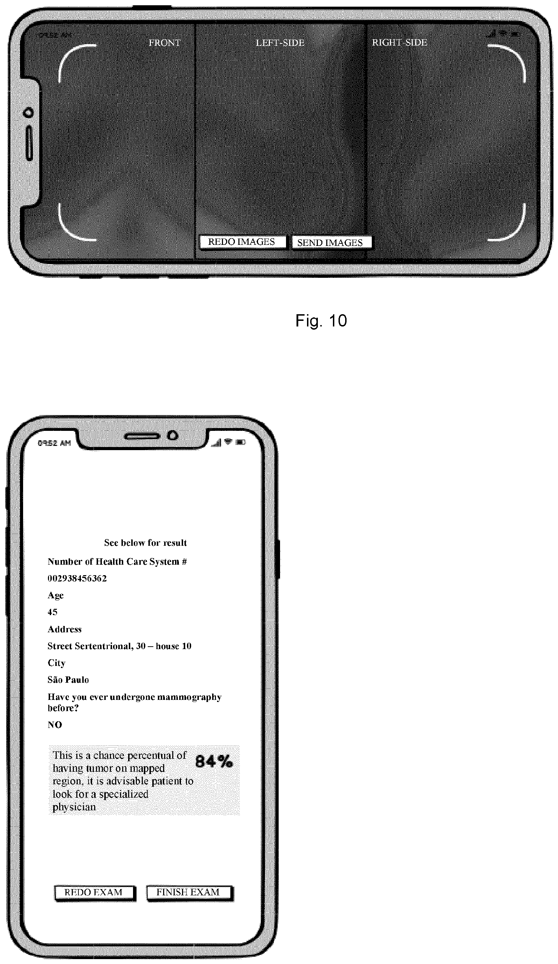

After capturing the frontal image, as illustrated in Fig. 7, the user reviews the captured image, judging if the image is good enough to be sent for evaluation in the artificial intelligence model. If the image is believed unsatisfactory, the user has the option to click on "Redo Image" to repeat the capture. If the image is believed satisfactory, the user clicks on "send image", submitting it for analysis by the artificial intelligence model. Optionally, the user can capture, in addition to the frontal image, the left and right-side images, as shown in figures 8 and 9, and with this the system can compose a single image formed by the three views, which is illustrated by figure 10. After the final composition of the image, the user judges the image according to the same process indicated above. In this case, the user has the option to choose whether to redo one of the three photos needed for the exam or redo all the images.

Also, the image review screen has integration with "Submission to the artificial intelligence model" - it sends image and patient data and returns a percentage of chance of having a pathological pattern.

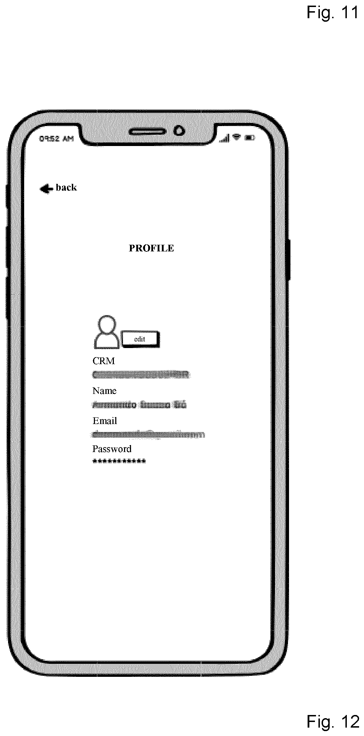

Fig. 11 shows the result screen. On the result screen, the app displays a summary with patient information and displays a percentage of the analysis performed by the artificial intelligence model. In addition, this percentage shows a text suggesting a next step to be followed by the user, such as, for example, that the user looks for a specialized physician if the percentage is high to positive. Also, clicking on "Redo Exam", the user is directed to the "Image capture (exam)" screen to redo the patient image. If the user clicks on "End Exam", they are directed to the "Pre-Exam" screen, which is the main screen of the application.

Also, the result screen has integration with "Save exam" - saves the exam as well as the image in the database and if there is a connection error, the user is warned and instructed to connect to a network. The app checks if there is an active internet connection.

Fig. 12 shows the profile screen. On this screen, the user views her registration data as well as edits her profile picture and changes the password to access the application. Also, the "back" function returns to the home screen.

Also, the profile screen has integrations with "Get user data" - the method brings the logged user data; and "Update User Data" - the user can change her photo or access password.

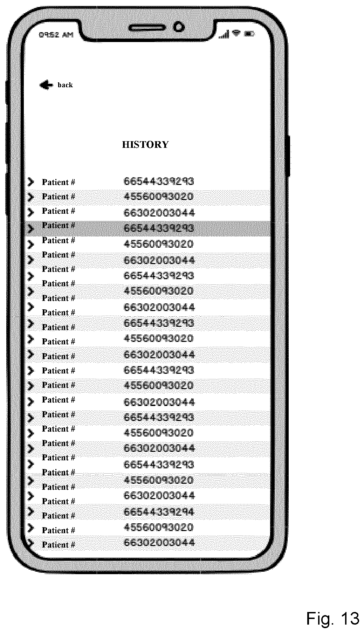

Fig. 13 shows the exam history screen. On this screen, the user views the list of exams performed by her. The history list is sorted by date, from newest to oldest. By scrolling through the history list, the application can continue loading the oldest historical exams, like an infinite list layout. Each line in the list displays the exam date and the patient number of the health care system. Clicking on a line in the list, the user is directed to the details screen of the clicked exam. At the top of the screen, the "back" button directs the user to the home screen.

Also, the exam history screen has integrations with "Get exam history" - method returns a list with the last 15 exams; and "Get More histories" - layout method that is called when the app reaches the end of the loaded list (15 exams).

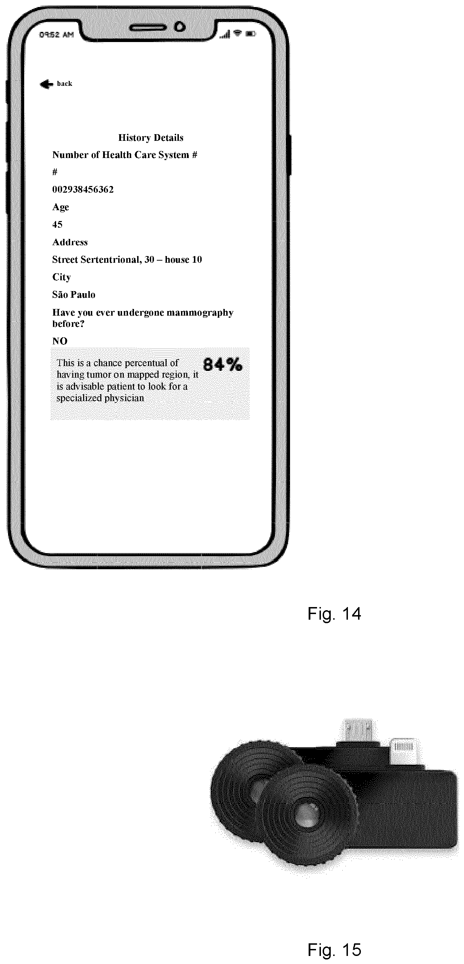

Fig. 14 shows the exam detail screen. On this screen, it is possible to review the patient information as well as the result of the analysis of the artificial intelligence model. At the top of the screen, the "back" button directs the user to the home screen.

Also, the exam detail screen has integration with "Get exam detail" - it returns the details of the selected exam, giving the patient number of health care system.

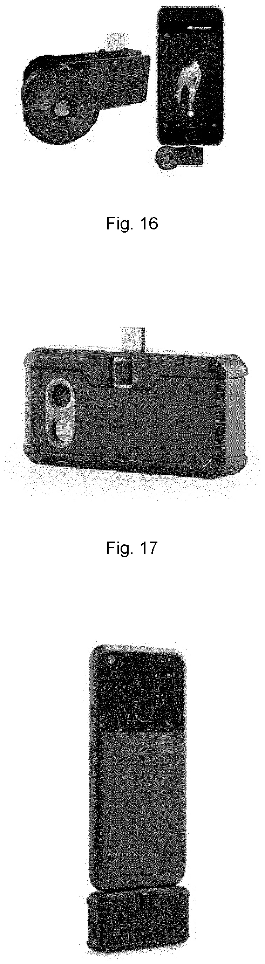

Fig. 15 shows an embodiment of the thermal camera and figure 16 shows said camera connected with a smartphone through physical connection.

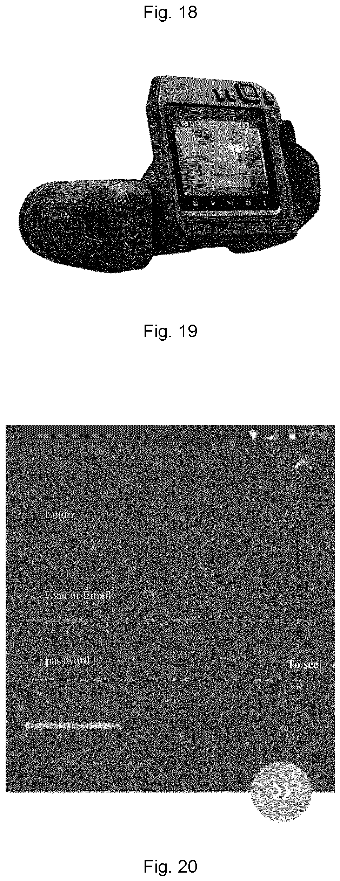

Fig. 17 shows another embodiment of the thermal camera and figure 18 shows said camera connected with a smartphone through physical connection.

Fig. 19 shows another embodiment of the thermal camera and figure 20 shows an app login screen, which shows that said thermal camera is connected to the smartphone via Wi-Fi.

Those skilled in the art will appreciate the knowledge presented herein and will be able to reproduce the invention in the modalities presented and in other variants and alternatives, covered by the scope of the claims below.

## Claims

1. Auxiliary mobile system for evaluating breast thermographic images, **characterized in that** it comprises:
a. a mobile electronic device connected with at least one thermal imager, the mobile electronic device being provided with at least one electronic application (3) embedded provided with an interface;
b. an intermediate module (2) communicating with the electronic application (3), the intermediate module (2) being provided with a data driver, in which the intermediate module (2) receives thermal image data from the electronic application (3); and
c. a server (1), communicating with the intermediate module (2), provided with an artificial intelligence tool and a data repository;
wherein,
- the intermediate module (2) sends the captured thermal image data to the server (1);
- the artificial intelligence tool receives the thermal image data and returns an index of suspicion, which is received by the intermediate module (2) and directed to the electronic application (3) embedded in the mobile electronic device; being the artificial intelligence tool previously trained and fed with breast thermographic images.

2. Auxiliary mobile system, according to claim 1, **characterized in that** the artificial intelligence tool comprises a convolutional neural network.

3. Auxiliary mobile system, according to claim 1, **characterized in that** the intermediate module (2) stores the index of suspicion received in the data repository of the server (1).

4. Auxiliary mobile system, according to claim 3, **characterized in that** the repository comprises a data history of at least one patient, wherein the history is updated with patient information and the index of suspicion.

5. Auxiliary mobile system, according to claim 1, **characterized in that** the electronic application interface (3) comprises at least one framework support mask in capturing the breast thermographic image.

6. Auxiliary process for evaluation of breast thermographic images **characterized in that** it comprises the steps of:
a. collecting at least one breast thermographic image by an electronic application (3) embedded in a mobile electronic device;
b. sending the breast thermographic image to a server (1), which is provided with an artificial intelligence tool evaluating the breast thermographic image and returning an index of suspicion; and
c. receiving the index of suspicion by the electronic application (3) and making available the index of suspicion in the interface of the electronic application (3).

7. Auxiliary process, according to claim 6, **characterized in that** it is implemented in an auxiliary mobile system, as defined in claim 1.

8. Auxiliary process, according to claim 6, **characterized in that** it further comprises a previous step of capturing the breast thermographic image by a thermal imager connected to the mobile electronic device, wherein the step of capturing the breast thermographic image comprises a sub-step of image framework from a support mask implemented in the interface of the electronic application (3).

9. Auxiliary process, according to claim 6, **characterized in that** the step of sending the breast thermographic image to a server (1) comprises the sub-steps of:
a. converting the breast thermographic image into a data string on the mobile electronic device; and
b. receiving evaluation request by an intermediate module (2), communicating with the electronic application (3) and with the server (1), wherein the intermediate module (2) receives the data from the breast thermographic image and directs it to the server (1);
wherein, the intermediate module (2) is responsible for receiving the index of suspicion evaluated by the artificial intelligence tool from the server (1) and sending it to the electronic application (3).

10. Auxiliary process, according to claim 6, **characterized in that** it further comprises a step of sending and updating patient history data, wherein the intermediate module (2) sends patient information and/or index of suspicion to the server repository (1).
